Europäisches Patentamt

European Patent Office

Office européen des brevets

(11) Publication number: **0 003 248**
A1

# EUROPEAN PATENT APPLICATION

(21) Application number: 78300613.3

(22) Date of filing: 08.11.78

(51) Int. Cl.²: **A 61 K 7/135**
C 11 D 3/39

(30) Priority: 09.11.77 NZ 185654

(43) Date of publication of application:
08.08.79 Bulletin 79/16

(84) Designated contracting states:
BE CH DE FR GB NL SE

(71) Applicant: Animark Products Limited
McLean Building 208 Oxford Terrace
Christchurch(NZ)

(72) Inventor: Lunn, Peter Frederick Raymond
Hoon Hay Road
Christchurch(NZ)

(72) Inventor: Grainger, Joyce
95 Dunns Avenue
Pines Beach Kaiapoi(NZ)

(74) Representative: Silverman, Warren et al,
HASELTINE LAKE & CO. Hazlitt House 28 Southampton
Buildings Chancery Lane
London WC2A 1AT(GB)

(54) Composition and method for bleaching hair or keratinous fibre.

(57) A bleaching system which is in powder form and capable on aqueous activation of bleaching hair or keratinous fibre in a simple one-stage procedure comprises as active ingredients a source capable on aqueous activation of generating persulphate ions and a percarbonate compound. The composition is particularly applicable for the marking of non-human animals which have a coat of hair or keratinous fibre and for this purpose, the composition may be rendered thixotropic by inclusion therein of pyrogenic silica.

EP 0 003 248 A1

Croydon Printing Company Ltd.

0003248

- 1 -

TITLE MODIFIED
see front page

Bleaching Systems

The invention relates to water activatable powders which include a persulphate and a percarbonate and which in the preferred form of the present invention upon water activation provide a thixotropic composition of viscocity suitable either for human hair bleaching or animal hair bleachmarking. The thixotropic agent of the powder is a pyrogenic silica. Preferred powder compositions also include a wetting agent, a sequestering agent and a humectant. The invention also consists in methods of bleaching and methods of application of such an activated bleach composition.

The present invention relates to bleaching compositions and in particular to relatively stable powder compositions capable of generating a wet bleaching composition. Preferably upon activation with water the composition generates a thixotropic aqueous solution which has application in the marking of dark haired animals and in particular spray marking. However such a system is also applicable in milder forms to the bleaching of human hair.

Many methods of marking or identifying animals are known. The traditional method has always been the use of heat branding. This results in a permanent marking which is hard to disguise, which has been good as a method of marking when there has been any tendency whatsoever to steal cattle etc. Such a method however, has two main disadvantages. The first is that the pelt of the animal is permanently damaged. The second is the fact that owing to the pain that necessarily follows the application of such a hot brand or the like, there is a period of, for example, one week following branding during which the animal gains no conditioning. This has severe economic ramifications when one considers the fact that for a vast number of animals annual marking is necessary. Other methods have been devised to overcome this. One that has led to some degree of success has been the use of cryogenic branding techniques, for example as exemplified in New Zealand Patent Specification No. 148,055. Such a system itself results in some permanent damage to the pelt although to a lesser extent than that caused by hot branding. The marking obtained from such cryogenic branding is as a consequence of the growth of white hair corresponding to the area frozen. However for effective marking using this method, it is necessary to prepare the surface of the skin to be treated, i.e. clipping or hair removal, first. Moreover such a marking is permanent and would not have great application in areas where, for example it is necessary to mark animals annually as a consequence of some testing procedure or the like. The most common form of marking that is used in order to identify individual animals and whether or not they have been subjected to a testing procedure is the use of paints or tags, e.g. ear tags. Ear tags, however, can fall out or be torn out as well as leading to considerable difficulties of identification from a distance owing to the size of the actual tag. It is believed that if one wrong number is taken off a tag during the course of culling of a group of animals, significant time is lost in subsequently trying to find the incorrectly numbered animal and also in checking whether or not the actual animal had in fact been tested etc. Paints or the like are either expensive or of limited effect in the sense that with long haired beasts the amount of material required is significant yet, can still be rubbed off. There is therefore some need to provide a system which will provide an effective and more economical method of marking which will overcome the abovementioned difficulties. Such a method, it is belived would involve the bleach marking of the hair of an animal. Normally the big moult of the year occurs in the spring. There is however, a secondary moult in autumn. One difficulty

- 3 -

0003248

however with the application of a bleach to a long haired beast is the requirement that there be penetration of the bleaching compoistion to the base of the hair in order for an effective mark to be provided. There is however the difficulty that good penetration accompanies a low viscosity of the bleaching compoisition. This however is not completely desirable owing to the fact that there can be run off which prevents the use of such a freely running composition in an effective manner to write numbers, if numbers are required. Obviously some applications of the bleaching composition would be to stripe each animal and to cross that stripe off once the results of testing or treating, for example for artificial insemination, brucellosis, TB or the like is known. However, there would be applications where large numbers would be desirable on the side or flank of each animal. It is believed therefore that if a sprayable composition is devised that has effective bleaching properties which do not significantly affect the well-being of the pelt of the animal and provided the same can be applied without a great deal of spill off that many of the shortcomings of existing methods would be forestalled and a new tool in the armoury of livestock rearers would result, thus making it practicable to deal with large numbers of animals in any testing or vetting procedure.

The use of hydrogen peroxide or other peroxide compositions as a bleaching agent has long been known. Hydrogen peroxide however, does have many disadvantages of which poor shelf life and difficulty of storage are the most important. When the same is used with human hair there is also a tendency for persons with sensitive scalps to be irritated by such use of hydrogen peroxide. More recently we have tested a mechanism has been utilised which provides an enhanced bleaching with a minimum of the hydrogen peroxide. This involves a two part mix of a persulphate with a source of peroxide ions with the actual bleaching mechanism merely being triggered by the presence of the peroxide ions. Even so, there is some desirability whereby it is not necessary to mix hydrogen peroxide with the bleaching agent.

Bleaching compositions have many uses, and one possible use is as a marking material for hair covered animals, such as cattle beasts. Here, it will be readily apparent that a one component bleach is most desirable, i.e. one to which water alone need be added instead of it being necessary to store and mix before use the somewhat unstable hydrogen peroxide and the bleaching composition to be triggered by the peroxide itself.

Two part mixes of hydrogen peroxide and a persulphate are taught in U.S. Patent Specification Nos. 3378444 and 1894277. A one part mix is disclosed in U.S. Patent Specification Nos. 1986672 and 3337466. Other disclosures of interest are contained in U.S. Patents 2254434, 2371545, 2914374, 2927082 and 3819828. U.S. Patent Nos. 3651931, 3679102 and 3931912 deal with various spraying techniques that can or do relate to bleaches.

However none has a shelf life or is effective bleaching condition considered satisfactory for both animal and human use. Moreover non satisfactorily provides a one component composition that is capable of being sprayed that is suitable for bleach marking of animals. Moreover no full appreciation has been given to the question of incorporating a thixotropic agent in the dry powder which will not effect the shelf life of the powder, yet will when in solution or aqueous suspension will enable the activated wet composition to be sprayed.

It is therefore an object of the present invention to provide a water activatable bleaching composition, and/or methods, formulations and/or techniques including marking techniques which relate to at least in part thereto.

In one aspect the present invention consists in a composition in powder form capable on aqueous activation of hair or keratinous fibre bleaching characterised in that it includes as active ingredients a source capable on aqueous activation of generating persulphate ions and a percarbonate compound. Preferably the percarbonate compound is sodium percarbonate although it could be potassium percarbonate. Preferably the source of persulphate ions is ammonium persulphate although it could be potassium persulphate.

- 5 -

0003248

Preferably from 25-60% by weight of the powder is ammonium persulphate and from 25-50% by weight of the powder is sodium percarbonate. Ideally any percarbonate present has a particle size of from 800 to 100 microns.

Ideally the composition when in its powder form also includes an agent capable of making any aqueous composition thereof thixotropic. For this purpose the thixotropic agent is preferably a pyrogenic silica and preferably that pyrogenic silica is present in from about 4 to about 20% by weight of the powder. Ideally also the powder includes at least one compatible agent selected from wetting agents, sequestering agents and humectant agents.

In a further aspect the invention consists in a composition in powder form for human or animal hair bleaching after water activation comprising pyrogenic silica, a soluble persulphate and an at least partially soluble percarbonate. Preferably said soluble persulphate is as before and preferably the percarbonate is as before.

In still a further aspect the present invention consists in a method of bleaching animal including human hair comprising steps of mixing a composition in powder form comprising a source of persulphate ions selected from ammonium and potassium persulphate and a percarbonate compound selected from sodium and potassium percarbonate with water to provide a wet mixture and applying the wet mixture to the hair to be bleached. Preferably the powder composition is as previously set forth and preferably the powder includes pyrogenic silica.

Preferably the wet mix has a viscosity greater than 200cp.

In still a further aspect the present invention consists in a method of marking a non human animal which has a coat of hair or keratinous fibre (e.g. a cattle beast) which comprises applying thereto a thixotropic alkaline bleaching composition formed by mixing water with a composition in powder form comprising a soluble persulphate and an at least partially soluble percarbonate. Preferably the powder composition is as previously set forth.

0003248

The invention also consists in a sealed marketable quantity of the powder.

Preferred forms of spray nozzle will now be described with reference to the accompanying drawings in which:

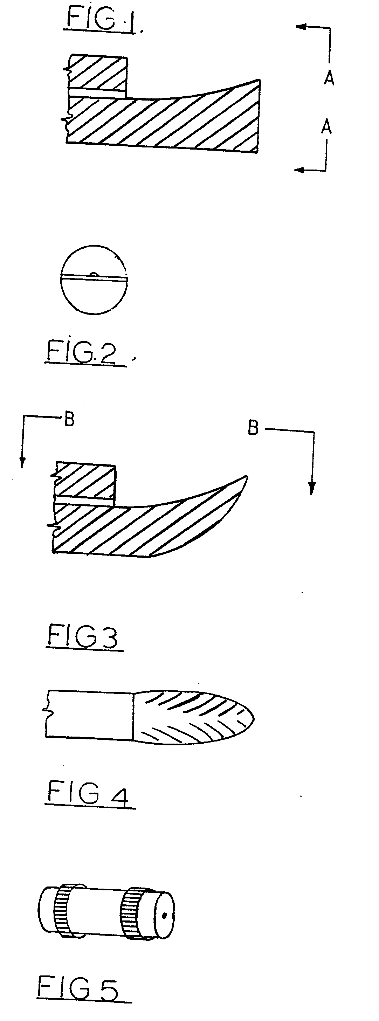

Figure 1 is a side elevation section of a preferred nozzle in accordance with the present invention,

Figure 2 is an end view AA of the same,

Figure 3 is a similar view to that shown in figure 1 but of a different form of nozzle in accordance with the present invention,

Figure 4 is a plan view BB of the nozzle shown in figure 3, and

Figure 5 shows a substantially standard nozzle suitable for figure marking.

The preferred form of the present invention will now be described with reference to the criteria that have been considered desirable for such a composition in order for such a composition or its availability to create a demand for the same as a sprayable marking composition. Basically however, it is convenient to consider the fact that many forms of livestock now have long hair, for example Pole Herefords, yet it is still necessary for these animals to be tested periodically (usually only once a year for brucellosis, TB or the like). It is also necessary for the same to be marked when in fact the same have been artificially inseminated. It is seen, therefore that a bleach mark would be most desirable since the same would be clearly visible for the duration of at least the period for which the test or treatment is applicable yet will be totally removed by the annual moult, or if not totally removed removed sufficiently so that confusion with a subsequent year's mark would not result. It is also envisaged that if required the application of the composition can be capable of being effected from a variable distance from the animal, for example, the most usual approximate distance from the animals up to 6" but in some cases the distance can be up to 18", yet will provide the animal, with, for example a straight mark which can subsequently be crossed off with a similar mark at right angles when in fact the test result of that animal is known

- 7 -

0003248

or some treatment has taken place. With such marking therefore the animals can be separated out and instantly at some subsequent time, for the currency of that marking, a farmer or any person interested can determine at a glance whether or not the animal has been tested or treated. It is also desirable of course to provide a sprayable bleaching composition that can be used to make large numbers or numbers of varying sizes on the animals. However, a consideration of the following history of the development of the novel and inventive composition will give an indication as to the difficulties that were confronted and which were subsequently overcome.

When the concept of bleach marking of animals was first considered by us, several types of bleaching composition that were effective yet did not unduly sting the skin of the animal to which it was applied were developed. Basically the original product was a powder which included a peroxygen compound which when mixed with hydrogen peroxide formed a gritty paste which could be applied by brush to the animal's hair. Subsequent to application, the bleaching effect triggered by the addition of the hydrogen peroxide resulted in the bleaching out of the natural pigment (melanin) thus yielding a servicable mark of a white or buff colour. With however, large numbers of animal the marking of animals with a brush is a slow and tedious task, especially when the animals have long hair. As an itial step towards developing a sprayable bleaching composition, a modification of the original composition was tried through an electric airless gun. However owing to the physical nature of the slurry of the bleach mix, the gun kept blocking. Other types of spraying equipment were then tried including pneumatic type sprays, but in all cases the sprays blocked and no method could be found which would dispense the formula effectively without the requirement for tedious brushing which would take the bleaching composition to the skin where the same would effectively bleach the whole hair. Early formulations contained so many insoluble substances, and it was believed that there was no way that such a bleaching composition could be made which would fulfill the requirements of a sprayable bleaching composition. It therefore became apparent that in order to produce a sprayable product the formulation would have to be changed. Difficulties in providing a sprayable composition were due largely to the inability to find a viscosity builder that would not block the spray. In every case the product after mixing would have had to have been strained into the spray container and mixed with meticulous care in order for the same to have some chance of being sprayed for a reasonable time period through the spraying mechanism of a spray gun. This obviously was totally unacceptable from the concept of marketing the product. At the same time it should also be appreciated that there was considerable difficulty in finding the right spray type of equipment, both from a capacity and function aspect and for this reason it took some considerable time to determine that a non-electric spray gun based on air as a propellant, which could be pumped by hand, would be the easiest to use especially under the difficult field conditions where such marking would take place, i.e. in the absence of electricity and of a ready source of compressed air. In order however, for the composition to be sprayed by such a spray gun it was necessary to prepare a bleaching powder mix that was almost entirely soluble in a peroxide developer and as an initial approach and for ease of spraying, it was decided to remove all viscosity builders. Many formulations were tried and finally a solution was formed with excellent bleaching properties to both animal and human hair and which could be maintained at a pH of above 9. Such a composition relied on persulphate and a peroxide. It was considered that urea peroxide was too dangerous to be marketed owing to its explosive nature even though it had some advantage in the sense that it is not liquid in form. For this safety aspect, it was considered that the source of the peroxide should be hydrogen peroxide despite its storage difficulties. It soon became apparent that one of the most important features for a good bleaching mix was that the pH of the product after mixing with the hydrogen peroxide solution should be kept as high as possible. Normally a hydrogen peroxide solution is highly unstable unless it is kept at an acidic pH and obviously any marketable product should if at all possible have the hydrogen peroxide maintained for long shelf life at an acidic pH, for example pH of 3 to 4. However, the mixing of an acidic solution of hydrogen peroxide with the powder of the two component mix ideally should yield a product with a highly alkaline resultant pH in order to provide effective bleaching. Hence it can be seen that the alkalinity of the powder is significantly lowered upon mixing with the peroxide and at one stage it was considered that it may be necessary to raise the pH of the hydrogen peroxide solution in order to overcome this difficulty. This could have however resulted in the shelf life of the hydrogen peroxide being significantly reduced especially in warmer climates. At that stage also, it was considered highly unlikely that an effective pH raising powder agent could be included in the powder component without subsequently upon mixing leading to sprayability difficulties.

The solution to this difficulty will be referred to hereinafter.

Since by this stage reasonably stable products had been chosen which would give a good bleaching effect when mixed with the hydrogen peroxide developer, attention was then turned to the problem remaining which was that the solution ran down the animal's coat and was inclined to look rather messy particularly when the person spraying the same was attempting to write a distinct number. An approach was therefore made to manufacturers of spray guns or the like to determine whether or not a special nozzle could be produced which would provide high penetrability of the animal's coat while at the same time spreading the same over a broad area. Two such spray nozzle types are illustrated hereinafter which a person skilled in the art of spray guns will recognise are ones that rely upon the orifice of the nozzle releasing the jet onto a sloped surface which fans the resultant spray in a flat and wide manner. Such sprays are ideal for providing a straight mark and also for providing lettering on the flank of the animal.

Reasons for choice of chemicals in the powder component at this stage:

## Oxidising Chemicals

The choice of ammonium persulphate was not because it was in an earlier formula, but because it produces the best bleaching effects and is the most soluble in solution. Sodium perborate has a poorer solubility, and has a lower bleaching effect. Probably the next best oxidising chemical to ammonium persulphate is potassium persulphate, and this was tried. Mainly because it has a higher pH, but though reasonably satisfactory, it still did not produce the optimum bleaching effects in the present medium, and is also not quite as soluble as the ammonium persulphate.

## pH Requirements

Many alkaline salts were tried. Some did not dissolve quickly enough, and others proved not to be sufficiently alkaline. Calcium oxide was tried in a two-fold attempt to raise the pH and form a sprayable slurry, but it caused the oxygen to be given off too quickly before depigmentation took place.

Probably the next best chemical to the final choice is sodium sesqui carbonate, but the most preferable chemical which was found to produce the highest alkalinity with the greatest degree of solubility is potassium carbonate.

## Wetting Agent

It was also necessary to have a wetting agent which dissolved quickly and easily in a cold solution without excessive mixing and without affecting the bleaching properties - few meet these requirements. The two different types of detergent that have been found most suitable are:

(i) A salt of N-lauryl beta-imino dipropionate (trade name DERIPHAT 160c).

(ii) A sodium salt of a sulphonated fatty acid, namely Sodium Lauryl Sulphate.

Both detergents should be in powder form.

## Sequestering Agent

The acid salt of ethylene diamine tetra acetic acid (E.D.T.A.) was chosen to chelate any metallic ions which may be present.

Once a sprayable mixture which produced a good bleaching effect was obtained it was decided to again examine the possibilities of thickening the solution to try to hold it in situ on the animal's coat. This would be particularly important where a series of numbers was required to be written on the animal.

Many thickening and/or thixotropic agents produce one or more of the following difficulties:

(i) Time taken to thicken the solution - it should be within 5 - 7 minutes. If the product stands too long after mixing with the $H_2O_2$ developer, it begins to deteriorate, and loses its bleaching properties - obviously this is an important factor in areas where the weather is very warm.

2) Some thickeners will not thicken in such an alkaline medium.

3) Some thickeners will only thicken if the solution is milled.

4) Unless rapidly and efficiently dispersed, some thickeners will form lumps which cause a blockage in the spray.

Examples of Viscosity Builders Used

| | |
|---|---|
| Sodium Alginate | Caused blockage - also tends to reduce shelf-life |
| Colloidal Aluminium Silicate | Requires milling or high speed mixing to form a gel. |
| Carboxy Methyl Cellulose | Slow swelling takes too long. |
| Methyl Cellulose | As above |
| Ethyl Cellulose | As above |
| Carboxy Vinyl Polymers | Formed lumps, blocked spray. |
| Silica Gel Microfined | Did not build viscosity even at 10% by weight of powder composition, and it was also mixed with magnesium trisilicate to try to form a sprayable slurry but was not satisfactory. |

The only viscosity builders found that will produce a satisfactory thixotropic gel which can be sprayed easily, do not block the spray, and adhere well to the animal's coat are thickeners described as being PYROGENIC SILICAS used

0003248

preferably in the range 10 to 20% by weight of the powder composition component. However broadest preferred range is 4-20% by weight of powder.

General Comments

The powder component is hygroscopic and must be packaged in a material which does not allow moisture to permeate through the pack.

It is mixed in the proportion of 1 part by weight powder to 3 parts by weight 25 volume hydrogen peroxide solution - this amount is not critical but is the best sprayable mixture. The volume of $H_2O_2$ we have been using is 25 volume (7½%). However 20 volume (6%) can be used and up to 40 volume (12%) though 40 volume is wasteful and unnecessary. We have also used a lower volume and find that a fairly satisfactory mark can be achieved using 7 volume $H_2O_2$. It is however found in cold weather that a volume lower than 18 volume would probably produce a much poorer bleaching effect. The dry powder formula for mixing with hydrogen peroxide has been tried under extremely adverse field conditions, where it was intermittently snowing or raining heavily, and the average temperature was somewhere around $2^{o}C$ - the results in these very poor conditions were very good.

PREFERRED FORMULA OF POWDER COMPONENT IF TO BE MIXED WITH HYDROGEN PEROXIDE

| | |
|---|---|
| Pyrogenic Silica | 4 - 20% by weight |
| e.g. Hoescht Trade Name Aerosil V.200 or, | |
| Cabot Corporation Trade Name Cab-O-Sil M7 | |

Wetting Agent

| | | | |
|---|---|---|---|
| Sodium salt of Sulphonated fatty acid | | | |
| (Powder_ or, a salt of N-lauryl Beta- | | | |
| iminodipropionate (Powder)_ | 1 - 4% | " | " |
| Sequestering Agent | 1% | " | " |
| Ammonium Persulphate | 25 - 60% | " | " |
| Potassium Carbonate | 25 - 53% | " | " |
| Humectant D-Glucitol | 0 - 1% | " | " |

- 13 -

0003248

The percentages referred to above in regard to the formula relate to the powder component only of the two-part mix. The percentages are by weight. Of course once the powder and hydrogen peroxide are mixed together the resultant solution would start to expand as the oxygen is evolved, this severely affecting shelf life.

However even with such a sprayable hydrogen peroxide activated composition marketing would be difficult owing to the need for a foolproof way whereby hydrogen peroxide could be stored for a long period prior to its being mixed with the powder component immediately before use. Therefore attention was next fixed on devising a water activatable sprayable composition which would take advantage of the experience derived from the development of a sprayable hydrogen peroxide activated composition. At the same time attention was also to be given to developing a system as spin off that is useful as a human hair bleach.

With hydrogen peroxide solutions the bottles of hydrogen peroxide can be bulky and difficult to store and unless the hydrogen peroxide is of a particular purity or is maintained at an acidic pH is is most suseptible to poor shelf life, especially where there are high temperatures. Moreover the solutions on opening the bottles can be most vunerable to contamination.

The line of research therefore was to try to produce a one packet product to which water only need be added. Since also the research was primarily directed to animal marking it was desirable that the resulting mixture upon the addition of water would be a sprayable gel with good bleaching effects. To obtain this one powder system one of the first chemicals considered was urea hydrogen peroxide but because of the unstable nature of urea hydrogen peroxide and the presence of certain inorganic as well as organic chemicals, it was realised that many difficulties would present themselves in the use of urea hydrogen peroxide in a mixture. Sodium peroxide was also tried but this created a violent giving off of the oxygen when the product was mixed with the water and the hydrogen was too quickly dissipated to bleach the

0003248

hair when the mixed composition was applied thereto. Moreover, sodium peroxide is a most unstable compound.

We then approached a manufacturer of urea hydrogen peroxide to see if it was possible to have the chemical encapsulated. This would have been one method of making the urea hydrogen peroxide safer to mix with other inorganic chemicals in the formula. We learnt however, that although encapsulation of this chemical was not impossible, it would be difficult to achieve economically and consequently this line of research was not pursued.

At this stage therefore, a two packet powder system was considered to be more feasible. With such a two pack system, one packet contains peroxygen compounds, thickeners, stablizers, etc and the other packet contains a powder which produced hydrogen peroxide when dissolved in water to a known volume (e.g. urea hydrogen peroxide). The two powders would be mixed together in a container and water added to the mixture. This system did produce a good bleaching effect on hair, was sprayable with a good viscosity, but we were unable to ensure that the urea hydrogen peroxide would have anything other than a very poor shelf life even when stored in optimum conditions. During the process of trying to produce a stable one powder system, we also tried experimenting with several oxidising agents in combination with one another to determine if it was possible to obtain better bleaching effects from a combination of peroxygen compounds in small percentages rather than relying on a large percentage of one peroxygen compound.

At the completion of these tests, it was decided that after using many different oxidising compounds, ammonium persulphate gave the best bleaching effect and had the greatest solubility in water. We also observed that when we had combined sodium percarbonate in the formula there seemed to be a triggering action taking place on the ammonium persulphate. Upon noting this, we again returned to the principal of a one powder system and from a series of experiments of combining ammonium persulphate and sodium percarbonate we found we were able to produce an extremely good bleaching effect by simply adding water to the mixed compound.

Other sources of peroxygen were tried such as sodium perborate but these were less effective and generally less soluble than the percarbonate.

Sodium percarbonate is preferable to other percarbonates such as potassium percarbonate as it is more readily obtainable in commercial quantities.

It was at this stage that we realised that the concept of producing a relatively stable one powder product which can be activated by adding water instead of hydrogen peroxide solution or the like and which can result in ⊏ high degree of depigmentation of hair or human hair was no⁻⁻ʒl, and of economic significance. At this stage we realised that any compatible soluble percarbonates and persulphates could be utilized.

We then determined the required ratios of ammonium persulphate to sodium persulphate. Once those ratios were determined for optimum results it was then necessary to find a gelling substance which would be compatible with the peroxygen compounds so that when the powder was mixed with water a thixotropic sprayable gel would be formed. This thixotropic sprayable gel property was considered desirable for the animal marking function of the composition, i.e. so as to minimise run off after application. All of the ingredients had to be freely soluble for this purpose in hot or cold water. Also a powder with such a thixotropic agent present must be able to have a reasonable shelf life when stored in moderately good conditions.

The gelling agent best suited to these conditions was a silica, which was preferably again of the pyrogenic type. Such a pyrogenic silica can be used in unusually high percentages to obtain the desired degree of consistency without in any way affecting the peroxygen compounds. In fact the same tends to enhance the stability of the same.

With the emphasis for sprayability being on solubility in water high concentrations of the thickener are required and for consistency of mix the entire contents of a packet or container of the bleaching composition powder should be used to ensure a consistent proportion of the thixotropic thickener with respect to the other dry components even if there is a tendency for the materials to settle out or stratify.

For human hair a thixotropic mix is not critical since application of the bleach is not normally by spraying. If the mix is not to be sprayed then solubility or fineness of dispersion is not essential. For example, microsized amorphous silica gel may be used. This has the advantage over a pyrogenic silica in that bulk quantities of the powder can be used in small lots without the likelihood of greatly varying mixes being obtained during the use of the bulk lot. A pyrogenic silica however should also be present, not so much for its thixotropic properties but rather for its property of making mix both dry and free running.

A wetting agent was also desirable to ensure that maximum contact in use between the bleaching components of the composition and the hair to be bleached. Preferred wetting agents were a salt of N-lauryl beta-iminodipropionate or a sodium salt of a sulphonated fatty acid. Here again the selection of wetting agent was such that the same would be freely soluble in cold or hot water.

A sequestering agent was also considered desirable and this agent must be compatible with the other chemicals so as not to interfere with the stability or effectivenss thereof. The requirement for a sequestering agent was to ensure that any metal ions present were kept in solution. Appropriate sequestering agents were an acidic salt of ethylene diamine tetra acetic acid, sodium ethylene diamine tetra acetic acid or mixtures thereof.

Many successful bleaching tests using such a composition were conducted on animal and human hair. A series of tests on animal hair was made in temperatures of 45°C (simulated) and in temperatures of 15 to 19°C. Marks were made under these temperature conditions every half hour for up to four hours after water activation of the power. The results of these temperature tests were excellent and the tests conducted at the high dry temperature proved that the material stayed active after mixing with the water for a minimum of 2½ hours before the marks started to show a lesser degree of continued bleaching, and even after four hours the material was still relatively active.

Under extremely cold conditions, which can slightly retard the bleaching process, it could be an advantage to use hot water instead of cold water.  Generally however for human hair hot water is better irrespective of temperature factors. Where hot water is not available, then in order to speed the bleaching process of animal hair the water could be substituted for by a hydrogen peroxide solution of 20 vol. (approximately 6%) or up to 40 vol. (approximately 12½%).  However, with human  hair, it is desirable to omit completely the hydrogen peroxide solution and in particular the higher volume peroxides that can be damaging to human hair.

The preferred powder of the present invention is hygroscopic and must be packaged in a suitable material which does not allow moisture to permeate through the pack.

The powder composition of the present invention is preferably mixed in the proportion of one part powder by weight to two parts of water by weight where a sprayable mixture is required.  For hand applications however, for example, the marking of horses where the mark should be as small and neat as possible and not run down the hair, then lesser proportions of water can be used.  Similarly, with human hair.

The preferred formulae of the present invention are therefore as follows:

FOR SPRAY MARKING

Pyrogenic Silica
e.g. Hoescht Trade Name Aerosil
V.200 or, Cabot Corporation Trade
Name Cabo-O-Sil M7                          4 - 20% by weight

Wetting Agent
Sodium Salt of sulphonated
fatty acid or a salt of
N-Lauryl Beta Iminodipropionate
(Powder)                                    1 - 4%  "        "

Sequestering Agent
Acid salt of ethylene diamine
tetra acetic acid or sodium
ethylene diamine tetra acetic
acid                                        0.5 - 2.5%  "      "

- 18 -

0003248

| | |
|---|---|
| Ammonium Persulphate | 25 - 60% by weight |
| Sodium Percarbonate | 25 - 50% " " |
| Humectant D-Glucitol | 0 - 1% " " |
| pH range | 8.8 - 10.5 |

In some forms the potassium salt of the persulphate and/or percarbonate can be used.

FOR HUMAN HAIR WHEN NOT TO BE APPLIED BY SPRAYING

| | |
|---|---|
| Pyrogenic Silica (e.g. Aerosil V.200) | 4.0 - 10.0% by weight |
| Microsized Amorphous Silica gel | 1.0 - 8.5% " " |
| Sodium Lauryl Sulphate | 3.0% " " |
| Ethylene Diamine Tetra Aceid Acid (or any recognised and compatible sequestering agent) | 1.0% " " |
| Potassium or Ammonium Persulphate | 35.0 - 50.0% " " |
| Potassium or Sodium Percarbonate | 35.0 - 50.0% " " |
| Dry Perfume | 0.25% " " |
| Protein | 0.25% " " |
| D-Glucitol | 0 - 1% " " |

pH range 8.8 - 9.2

The proportions referred to above refer solely to the powder mix.

For best mixing the percarbonate should be readily dispersible especially if not very soluble. For this purpose a particle size of about 800-1000 microns is best. Similarly for best sprayability yet anti-run two parts by weight of water to the powder should be used and the viscosity in solution or suspension should be at least 200cp.

The sprayable bleaching composition when finally mixed has the advantage that the same upon swirling in the spray gun becomes sufficiently thin so as to be sprayed i.e. exhibiting its true thixotropic nature. Whereupon it is possible to spray neat letters onto the animal and once the bleaching composition as been sprayed onto the animal the same upon standing almost immediately resumes the higher viscosity which tends to prevent the same dripping down the animal from where it has been applied to the skin and hair of the animal.

From the foregoing then it can be seen that the present invention provides reasonably stable powder compositions

0003248

which are capable of being blended with water or for that matter if necessary hydrogen peroxide solutions to provide an effective sprayable bleaching composition. Moreover the present invention provides novel marking techniques and methods of bleaching. We believe therefore that the compositions, systems and techniques of the present invention will find widespread commercial use.

0003248

CLAIMS:

1.    A composition in powder form capable on aqueous activation
of hair or keratinous fibre bleaching characterised in that
it includes as active ingredients a source capable on
aqueous activation of generating persulphate ions and a
percarbonate compound.

2.    A composition as claimed in claim 1 wherein said source
of persulphate ions is ammonium persulphate and/or potassium
persulphate.

3.    A composition as claimed in claim 1 or claim 2 wherein
said percarbonate compound is sodium percarbonate and/or
potassium percarbonate.

4.    A composition as claimed in claim 3 wherein from about
25-60% by weight of the powder is ammonium persulphate and
from about 25-50% by weight of the powder is sodium percarbonate.

5.    A composition as claimed in claim 3 or 4 wherein said
sodium percarbonate has a particle size of from about 800 to
about 1000 microns.

6.    A composition as claimed in any one of the preceding
claims wherein said powder also includes an agent capable of
making any aqueous composition thixotropic.

7.    A composition as claimed in claim 6 wherein said agent
is a pyrogenic silica.

8.    A composition as claimed in claim 7 wherein said pyrogenic
silica is present in from about 4 to about 20% by weight of
the powder.

9.   A composition as claimed in claim 1 wherein said powder includes at least one compatible agent selected from wetting agents, sequestering agents and humectant agents.

10.   A composition as claimed in claim 3 comprising:

| | | |
|---|---|---|
| Pyrogenic silica | 4 - 20% | by weight/weight of powder |
| Wetting agent (a salt of N-Lauryl Beta Iminodipro-pionate) | 1 - 4% | " |
| Sequestering agent (selected from an acid salt or ethylene diamine tetra acetic acid and sodium ethylene diamine tetra acetic acid) | 0.5 - 2.5% | " |
| Ammonium Persulphate | 25 - 60% | " |
| Sodium Percarbonate | 25 - 50% | " |
| Humectant D-Glucitol | 0 - 1% | " |

having when activated a pH of from about 8.8 to about 10.5.

11.   A composition as claimed in claim 1 comprising:

| | | |
|---|---|---|
| Pyrogenic silica | 4.0 - 10.0% | by weight/weight of powder |
| Microsized Amorphous Silica gel | 1.0 - 8.5% | " |
| Sodium Lauryl Sulphate | 3.0% | " |
| Sequestering Agent | 1.0% | " |
| Percarbonate (selected from Potassium and Sodium percarbonate) | 35.0 - 50.0% | " |
| Dry Perfume | 0.25% | " |
| Protein | 0.25% | " |
| D-Glucitol | 0 - 1% | " |

pH range 8.8 to 9.2

0003248

12. A method of bleaching animal including human hair comprising the steps of mixing a composition in powder form as claimed in any one of the preceding claims with water to provide a wet mixture and applying the mixture to the hair to be bleached.

13. A method as claimed in claim 6 hwerein the viscosity of the wet mix is greater than 200cp and the wet mix has a pH from about 8.8 to about 10.5.

14. A method of marking a non-human animal which has a coat of hair or keratinous fibre which comprises applying thereto for a sufficient time a thixotropic alkaline bleaching composition formed by mixing a composition as claimed in any one of claims 1 to 11 and water.

15. A method as claimed in 14 wherein said bleaching composition is applied by spraying the composition onto the animal.

17. A marketable unit comprising a vapour proof container, package, bag or box of a composition as claimed in any one of claims 1 to 11.

1/1

0003248

FIG.1.

FIG.2.

FIG 3

FIG 4

FIG 5

European Patent Office

# EUROPEAN SEARCH REPORT

0003248

Application number

EP 78 30 0613

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| X | CHEMICAL ABSTRACTS, vol. 78, no. 8 February 26, 1973, Columbus, Ohio, USA S. YAMAMOTO et al.: "Cosmetic bleach" <br><br> & JP - A - 71 43560 (SUNSTAR) <br> * Page 268, abstract no. 47667y * | 1-15, 17 | A 61 K 7/135 <br> C 11 D 3/39 |
| X | FR - A - 998 485 (S.E.R.) <br> * Entirely * | 1-15, 17 | |
| | | | TECHNICAL FIELDS SEARCHED (Int.Cl.²) |
| X | FR - A - 940 799 (LAB. SCI. DE NEUILLY) <br><br> * Entirely * | 1-15, 17 | A 61 K 7/135 <br> C 11 D 3/39 |
| X | FR - A - 2 317 910 (MINNESOTA MINING) <br><br> * Page 1, line 20 - page 3, line 26; page 4, line 25 - page 5, line 5; page 8, lines 6-40; the examples; the claims * | 1-15, 17 | |
| X | DE - A - 1 926 434 (FMC) <br><br> * Page 2, lines 11-25; page 4, lines 1-12; the claims * | 1-15, 17 | CATEGORY OF CITED DOCUMENTS <br> X: particularly relevant <br> A: technological background <br> O: non-written disclosure <br> P: intermediate document <br> T: theory or principle underlying the invention |
| A | US - A - 3 823 231 (BUCARIA) | 1 | E: conflicting application |
| A | CH - A - 465 114 (TINTEX) | 1 | D: document cited in the application |
| A | DE - A - 2 432 614 (KINTEX) | 1 | L: citation for other reasons |
| A | FR - A - 1 411 692 (SCHENLEY) ./. | 1 | &: member of the same patent family, corresponding document |

X | The present search report has been drawn up for all claims

| Place of search | Date of completion of the search | Examiner |
|---|---|---|
| The Hague | 08-02-1979 | JONAS |

EPO Form 1503.1   06.78

European Patent Office

**EUROPEAN SEARCH REPORT**

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| A | FR - A - 1 311 807 (NOVAG) | 1 | |
| | ---- | | |

TECHNICAL FIELDS
SEARCHED (Int. Cl.²)